# EUROPÄISCHE PATENTSCHRIFT

(11) **EP 3 209 349 B1**
(45) Veröffentlichungstag und Bekanntmachung des Hinweises auf die Patenterteilung: **14.08.2019**
(21) Anmeldenummer: 15787461.1
(22) Anmeldetag: 22.10.2015
(51) Int. Cl.: A61M 1/16, G05B 23/00

(54) **SYSTEM UMFASSEND MEHRERE MEDIZINISCHE GERÄTE**
SYSTEM COMPRISING A PLURALITY OF MEDICAL DEVICES
SYSTÈME COMPRENANT PLUSIEURS ÉQUIPEMENTS MÉDICAUX

(30) Priorität: 24.10.2014 DE 102014015795
(43) Veröffentlichungstag der Anmeldung: 30.08.2017
(73) Patentinhaber: Fresenius Medical Care Deutschland GmbH, 61352 Bad Homburg (DE)
(72) Erfinder: MAIERHOFER, Andreas, 97422 Schweinfurt (DE)
(74) Vertreter: Herrmann, Uwe
(86) Internationale Anmeldenummer: PCT/EP2015/002093
(87) Internationale Veröffentlichungsnummer: WO 2016/062405

(56) Entgegenhaltungen:
- EP-A1- 0 613 688
- EP-A1- 2 716 308
- WO-A1-2012/166377
- DE-A1- 19 933 223

## Beschreibung

Die Behandlung von Dialysepatienten findet typischerweise in Dialysezentren statt, wobei mehrere Dialysegeräte an dieselben Versorgungsquellen (z.B. für Strom, Wasser, Konzentrat, etc.) angeschlossen sind und wobei die Dialysegeräte unter denselben Umgebungsbedingungen (z.B. Temperatur, Luftfeuchtigkeit, Helligkeit, etc.) betrieben werden. In den einzelnen Dialysegeräten werden durch diverse Aktoren (z.B. Pumpen, Mischsysteme, Spannungsregler, etc.) bestimmte Bedingungen (z.B. hinsichtlich elektrischer Spannung, Stoffkonzentration in der Flüssigkeit, Temperatur, etc.) gemäß geräteseitiger Vorgaben oder gemäß Benutzervorgaben eingestellt.

An geeigneten Stellen befinden sich im Dialysegerät ferner Sensoren, die Messwerte (z.B. elektrische Spannung, Leitfähigkeit, Temperatur, etc.) liefern. Für einige dieser Messwerte kann auf der Grundlage bekannter Eigenschaften der Versorgungsquellen und auf der Grundlage geräteseitiger Vorgaben bzw. Benutzervorgaben ein Erwartungswert angegeben werden.

Weicht ein Messwert zu weit von einem Erwartungswert ab, kann auf einen Fehler geschlossen werden. Die Abweichung und folglich der Fehler können allerdings mehrere Ursachen haben. Beispielsweise kann die Ursache in einer Fehlfunktion eines Aktors oder Sensors eines einzelnen Geräts liegen. Ferner kann die Ursache in einem Fehler an der Versorgungsquelle liegen, die an mehrere Geräte angeschlossen ist.

Um adäquat auf einen Fehlerzustand reagieren zu können (Sicherstellung der optimalen Funktion des Dialysegeräts, Erkennung potentieller Gefahren für den Patienten), ist es wünschenswert, zwischen möglichen Ursachen unterscheiden zu können.

Im Stand der Technik ist es bekannt, kritische Sensoren in Dialysegeräten redundant auszulegen bzw. die Möglichkeit zu schaffen, über spezielle Abgleichschaltungen den Messwert eines Sensors mit dem eines anderen gleichartigen Sensors zu vergleichen. Dies erfordert einen zusätzlichen konstruktiven Aufwand. Um "common mode"-Fehler zu vermeiden, muss im Prinzip die gesamte Messkette vom Sensor bis zur auswertenden Elektronik unabhängig konstruiert sein, was oft nur mit hohem Aufwand möglich ist. Auch sind Aussagen über den Zustand gemeinsamer Versorgungsquellen vieler Geräte kaum möglich, da sich die bereitgestellten Mittel (z.B. Flüssigkeit, elektrische Spannung, etc.) auf dem Weg zu einem einzelnen Gerät verändern können.

Dialysesysteme mit mehreren Dialysegeräten und einem gemeinsamen Versorgungssystem sind z.B. aus EP2716308 (A1) und WO2012/166377 (A1) bekannt.

Aufgabe der Erfindung ist es, eine Möglichkeit bereitzustellen, Ursachen für die Abweichung eines Messwerts von einem Erwartungswert feststellen zu können, wobei die Nachteile des Standes der Technik vermieden werden. Der vorliegenden Erfindung liegt alternativ oder zusätzlich die Aufgabe zugrunde, eine Möglichkeit bereitzustellen, Ursachen für die Abweichung einer oder mehrerer Geräteeinstellungen und/oder für die Abweichung einer oder mehrerer Benutzervorgaben von einem Erwartungswert feststellen zu können.

Die Erfindung wird durch die Merkmale des unabhängigen Anspruchs 1 definiert. Vor diesem Hintergrund betrifft die Erfindung ein System umfassend mehrere medizinische Geräte, die an ein gemeinsames Versorgungssystem angeschlossen sind, wobei vorzugsweise vorgesehen ist, dass die Geräte jeweils wenigstens einen Sensor zur Ermittlung eines Messwertes aufweisen. Erfindungsgemäß ist vorgesehen, dass das System eine Auswerteeinheit aufweist, die mit allen Geräten des Systems in Verbindung steht und so ausgebildet ist, dass in den unterschiedlichen Geräten eingestellte Werte und/oder vorgegebene Werte und/oder ermittelte Messwerte, die sich auf korrespondierende Größen bzw. Messgrößen beziehen, zur Erkennung von Fehlern des Versorgungssystems und/oder eines einzelnen Geräts mit wenigstens einem Erwartungswert verglichen werden.

Erfindungsgemäß werden somit Messwerte und/oder (Geräte-) Einstellungen, d.h. eingestellte Werte, wie z.B. Flussraten der Blutpumpe, der Dialysatpumpe etc., und/oder (Benutzer-) Vorgaben, d.h. vorgegebene Werte, wie z.B. ein UF-Profil etc. mit wenigstens einem Erwartungswert verglichen. Die Messwerte, die eingestellten Werte sowie die vorgegebenen Werte werden im Rahmen der Erfindung auch einfach als "Werte" bezeichnet. Der Begriff "Werte" kann somit ein oder mehrere Messwerte und/oder eingestellte Werte und/oder vorgegebene Werte umfassen.

Das System umfasst vorzugsweise mindestens drei Geräte, wobei vorgesehen sein kann, dass das System mehr als fünf oder zehn Geräte umfasst. Es kann vorgesehen sein, dass mehrere oder alle Geräte des Systems dieselbe Bauart oder unterschiedliche Bauarten aufweisen.

Vorzugsweise handelt es sich bei den medizinischen Geräten um Dialysegeräte, wobei von der Erfindung auch Systeme umfassend mehrere andere medizinische Geräte, beispielsweise Apheresevorrichtungen umfasst sind. Als Dialysegeräte werden Geräte zur Durchführung einer Hämodialyse, Hämofiltration, oder Hämodiafiltration oder eines ähnlichen Blutreinigungsverfahrens verstanden.

Bei dem gemeinsamen Versorgungssystem kann es sich beispielsweise um ein zentrales System zur Bereitstellung von elektrischer Energie und/oder von gereinigtem Wasser und/oder von Dialyseflüssigkeits-Konzentrat und/oder von Dialyseflüssigkeit handeln.

Bei den korrespondierenden Größen oder Messgrößen kann es sich um identische Größen oder Messgrößen oder rechnerisch miteinander in Verbindung stehende Größen bzw. Messgrößen handeln. Handelt es sich bei den Werten um Messwerte, können auch diese unter Verwendung mehrerer Sensoren ermittelt werden (z.B. "blood-temperature-monitoring" (BTM)).

Insbesondere kann die Messgröße mit Hilfe einer Vorrichtung ermittelt werden, die die Messgröße mittels eines oder mehrerer Sensoren, ggf. nach Änderung der Behandlungsparameter durch geeignete Aktoren durch eine Auswerteeinheit bestimmt.

Die Sensoren können beispielsweise Leitfähigkeits- und Flusssensoren sein, der Aktor kann beispielsweise zur Änderung der Dialysatzusammensetzung vorgesehen sein. Mit diesen Sensoren und diesem Aktor kann eine Bestimmung der Diaylseeffizienz erfolgen (OCM: "online clearance monitor").

Bei den Sensoren kann es sich auch um Temperatur- und Flusssensoren handeln und bei dem Aktor um einen solchen, der zur Änderung der Dialysattemperatur dient. Die Vorrichtung kann entsprechend eine Vorrichtung zur Bestimmung der Rezirkulation sein.

Die Sensoren zur Ermittlung der korrespondierenden Messgrößen können in mehreren Geräten des Systems dieselbe oder unterschiedliche Bauarten bzw. dasselbe oder unterschiedliche Funktionsprinzipien haben.

Bei dem Erwartungswert kann es sich um einen gerätespezifischen oder einen geräteunabhängigen Erwartungswert handeln. Ein gerätespezifischer Erwartungswert kann beispielsweise dann angenommen werden, wenn der Erwartungswert von den patientenspezifischen Einstellungen am Gerät abhängt. Ein geräteunabhängiger Erwartungswert kann beispielsweise dann angenommen werden, wenn der Erwartungswert für alle Geräte derselbe ist und nicht von einer beispielsweise patientenspezifischen Einstellung am Gerät abhängt. Der geräteunabhängige Erwartungswert kann auf einer für das betreffende Behandlungszentrum, z.B. Dialysezentrum typischen Einstellung an den Geräten beruhen.

In einer Ausführungsform ist die Auswerteeinheit so ausgebildet, dass der Erwartungswert ein Mittelwert mehrerer sich auf die korrespondierenden Größe / Messgröße beziehender Werte bzw. Messwerte ist. Die beispielsweise arithmetische Mittelwertbildung kann über die zu einer bestimmten Größe / Messgröße gehörenden Werte / Messwerte aller Geräte erfolgen. Ferner ist denkbar, dass der Wert / Messwert des zu untersuchenden Gerätes ausgeklammert wird, und die Mittelwertbildung über die zu der bestimmten Größe / Messgröße gehörenden Werte / Messwerte aller verbleibenden Geräte erfolgt.

In einer Ausführungsform ist die Auswerteeinheit so ausgebildet, dass es als Fehler des Versorgungssystems identifiziert wird, wenn zu korrespondierenden Größen bzw. Messgrößen gehörige Messwerte oder sonstige Werte mehrerer oder aller Geräte des Systems von dem oder den Erwartungswerten vorzugsweise um etwa denselben Betrag und/oder mit demselben Vorzeichen abweichen.

Die Auswerteeinheit ermittelt also anhand einer bestimmten im Algorithmus der Auswerteeinheit hinterlegten Regel, ob ein vom Erwartungswert abweichender Messwert oder sonstiger Wert auf ein einzelnes Gerät beschränkt ist (vermutlich liegt dann ein Fehler in dem betroffenen Gerät vor) oder ob er in mehreren Geräten auftritt (vermutlich liegt dann ein Fehler im Versorgungssystem vor).

Beispielsweise kann es als Fehler des Versorgungssystems identifiziert werden, wenn zu korrespondierenden Größen, wie z.B. Messgrößen gehörige Messwerte oder andere Werte in Form von Benutzervorgaben oder Geräteeinstellungen von zwei, von mehr als zwei oder von allen Geräten des Systems von dem bzw. den Erwartungswerten abweichen. Dabei kann ggf. auch berücksichtigt werden, ob das Schema der Abweichung vom Erwartungswert jeweils ähnlich ist, etwa ob die Abweichung denselben Betrag und/oder dasselbe Vorzeichen hat.

In einer Ausführungsform ist die Auswerteeinheit so ausgebildet, dass es als Fehler eines einzelnen Gerätes identifiziert wird, wenn nur ein oder lediglich vereinzelte zu korrespondierenden Messgrößen oder sonstige Größen gehörige Messwerte bzw. Werte von dem oder den Erwartungswerten abweichen.

Die Auswerteeinheit ermittelt also anhand einer bestimmten im Algorithmus der Auswerteeinheit hinterlegten Regel, ob für ein bestimmtes Gerät ein vom Erwartungswert abweichender Messwert oder sonstiger Wert vorliegt, und schließt auf dieser Grundlage auf einen Fehler in dem betroffenen Gerät.

Es kann vorgesehen sein, dass Abweichungen vom Erwartungswert nur dann beachtet werden, wenn der Messwert oder sonstige Wert den Erwartungswert in gewissem Ausmaß, z.B. um mehr als 5% oder 10% übersteigt bzw. unterschreitet.

In einer Ausführungsform ist die Auswerteeinheit so ausgebildet, dass eine Trendanalyse der Vergleichsdaten vorgenommen wird, anhand welcher Drifts im Versorgungssystem und/oder einzelner Sensoren erkannt werden.

Die Trendanalyse erfolgt über einen bestimmten Zeitraum, beispielsweise während einer Behandlung oder über mehrere Behandlungen hinweg. Anhand einer derartigen Analyse können Langzeit-Drifts im Versorgungssystem oder Langzeit-Drifts einzelner Sensoren erkannt werden. Bei derartigen Drifts handelt es sich um Unterfälle von Fehlern im Sinne der vorliegenden Erfindung.

In einer Ausführungsform ist die Auswerteeinheit so ausgebildet, dass es als Drift des Versorgungssystems identifiziert wird, wenn sich Abweichungen zu korrespondierenden Messgrößen bzw. Größen gehöriger Messwerte bzw. Werte mehrerer oder aller Geräte des Systems von dem bzw. den Erwartungswerten mit der Zeit erhöhen.

Die Auswerteeinheit ermittelt also anhand einer bestimmten im Algorithmus der Auswerteeinheit hinterlegten Regel, ob zu korrespondierenden Messgrößen bzw. sonstigen Größen gehörige Messwerte oder sonstige Werte von zwei, von mehr als zwei oder von allen Geräten des Systems mit der Zeit immer mehr von einem oder mehreren Erwartungswerten abweichen. Dies legt die Vermutung eines Drifts des Versorgungssystems nahe. Dabei kann ggf. auch berücksichtigt werden, ob das Schema der Entwicklung der Abweichung jeweils ähnlich ist, etwa ob in allen Fällen eine Zunahme oder Abnahme des Messwertes / Wertes vorliegt.

In einer Ausführungsform ist die Auswerteeinheit so ausgebildet, dass es als Drift eines einzelnen Gerätes identifiziert wird, wenn sich die Abweichungen nur eines oder lediglich vereinzelter zu korrespondierenden Messgrößen / Größen gehöriger Messwerte oder sonstiger Werte von dem bzw. den Erwartungswerten mit der Zeit erhöhen.

Die Auswerteeinheit ermittelt also anhand einer bestimmten im Algorithmus der Auswerteeinheit hinterlegten Regel, ob für ein bestimmtes Gerät ein Erwartungswert mit der Zeit immer mehr vom Mittelwert abweicht, und schließt auf dieser Grundlage beispielsweise auf einen Drift des betroffenen Sensors in dem betroffenen Gerät.

In einer Ausführungsform handelt es sich bei dem Messwert oder sonstigen Wert im Sinne einer Geräteeinstellung oder Nutzervorgabe um die Leitfähigkeit der Dialyseflüssigkeit vorzugsweise stromaufwärts des Dialysators, und der Vergleich dient zur Erkennung von Fehlern einer zentralen Konzentrat-Versorgung, einer zentralen Dialyseflüssigkeitsversorgung, einer zentralen Reinwasserversorgung, einer gerätespezifischen Dialyseflüssigkeits-Mischanordnung oder eines gerätespezifischen Leitfähigkeits-Sensors.

In einer Ausführungsform handelt es sich bei dem Messwert oder sonstigen Wert um die Leitfähigkeit, den Geräteeingangsdruck oder die Geräteeingangstemperatur des RO-Wassers, und der Vergleich dient zur Erkennung von Fehlern einer zentralen RO-Wasser-Versorgung.

In einer Ausführungsform handelt es sich bei dem Messwert oder sonstigen Wert um den absoluten Wert oder die Varianz der Versorgungsspannung, und der Vergleich dient zur Erkennung von Fehlern in der zentralen oder gerätespezifischen Stromversorgung.

Geeignete Sensoren zur Bestimmung des Messwertes umfassen ferner Temperatursensoren, optische Sensoren (z.B. Blutdetektoren), oder Ultraschallsensoren. Die Sensoren können an die Geräte nachgerüstet werden oder daran vorinstalliert sein.

Weitere spezifische Anwendungen ergeben sich aus den Ausführungsbeispielen.

In einer Ausführungsform umfasst das System ein zentrales Anzeigemittel und/oder Anzeigemittel an den Geräten. Fehler des Versorgungssystems und/oder eines einzelnen Sensors können zentral und/oder einzeln an den Geräten des Systems angezeigt werden. Beispielsweise kann vorgesehen sein, dass ein Fehler des Versorgungssystems zentral und/oder an allen Geräten angezeigt wird. Ein Fehler eines einzelnen Gerätes kann vornehmlich am betroffen Gerät selbst und ggf. ferner zentral angezeigt werden, jedoch nicht an den nicht betroffenen Geräten.

Die Offenbarung betrifft ferner ein nicht beanspruchtes Verfahren zum Betreiben eines Systems umfassend mehrere medizinische Geräte, vorzugsweise Dialysegeräte, die an ein gemeinsames Versorgungssystem angeschlossen sind, wobei vorzugsweise vorgesehen ist, dass die Geräte jeweils wenigstens einen Sensor zur Ermittlung eines Messwertes aufweisen. Erfindungsgemäß werden in unterschiedlichen, in Betrieb befindlichen Geräten des Systems eingestellte Werte und/oder vorgegebene Werte und/oder ermittelte Messwerte, die sich auf korrespondierende Messgrößen bzw. sonstige Größen beziehen, zur Erkennung von Fehlern des Versorgungssystems und/oder eines einzelnen Gerätes mit wenigstens einem Erwartungswert verglichen.

Vorteilhafte Ausgestaltungen ergeben sich aus dem in der Auswerteeinheit des erfindungsgemäßen Systems hinterlegten Algorithmus.

Weitere Einzelheiten und Vorteile der Erfindung ergeben sich aus den nachfolgend beschriebenen Figuren und Ausführungsbeispielen. In den Figuren zeigen:
- Figur 1:: eine schematische Darstellung der Struktur eines erfindungsgemäßen Systems;
- Figur 2:: eine schematische Darstellung eines Dialysegerätes mit Eignung zur Anwendung in einem erfindungsgemäßen System; und
- Figur 3:: Graphiken von Abweichungsszenarien der Leitfähigkeit der Dialyseflüssigkeit, die im Rahmen des erfindungsgemäßen Systems ausgewertet werden können.

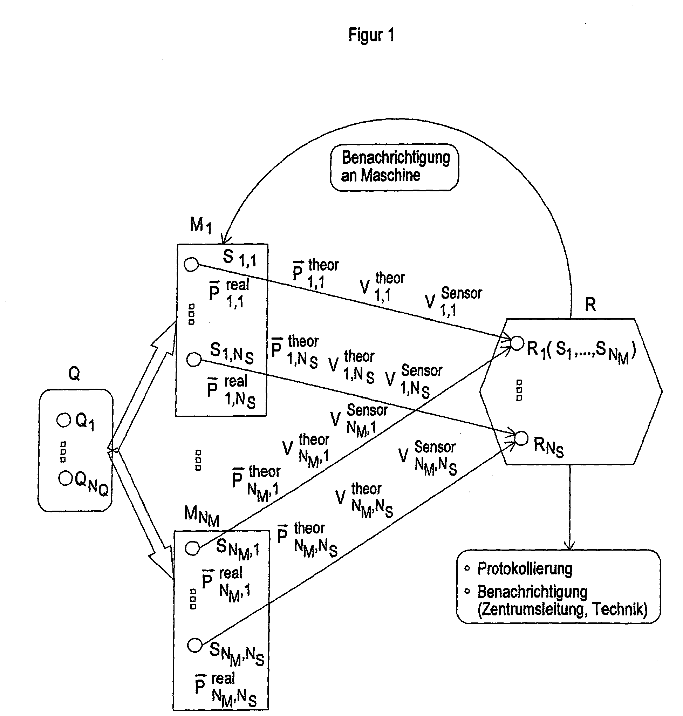

Figur 1 zeigt eine schematische Darstellung der Struktur eines erfindungsgemäßen Systems.

Die Dialysegeräte M₁ bis M_{nM} sind mit den Versorgungsquellen Q₁ bis Q_{nQ} verbunden. In jedem Dialysegerät *j* befinden sich die Sensoren S_{j,1} bis S_{j,nS}. Dabei können sich mehrere Sensoren an einem Ort befinden. An jedem Sensor k liegen real die Umgebungsbedingungen P^{real}_{j,k} = (p₁, p₂, ..., p_{nP}) vor, wobei p₁ bis p_{nP} beliebige Eigenschaften wie beispielsweise Temperatur, Konzentration eines Stoffes in der Lösung, Drücke etc. bezeichen.

Diese realen Eigenschaften sind jedoch unbekannt, vielmehr existieren über sie die Annahmen P^{theor}_{j,k} = (p₁, p₂, ..., p_{nP}) auf Grund der Einstellungen im Dialysegerät und den Annahmen über die Eigenschaften der Versorgungsquellen. Basierend auf diesen Annahmen wird im Dialysegerät mittels eines hinterlegten Modells ein Erwartungswert V^{theor}_{j,k} = *f*(P^{theor}_{j,k}) für den Messwert des Sensors·berechnet. Real misst dieser Sensor aber den Wert V^{real}_{j,k} = *f*(P^{real}_{j,k}). Für alle Sensoren S_{j,k} aller aktiven Dialysegeräte Mⱼ werden die Werte von P^{theor}_{j,k}, V^{theor}_{j,k} und V^{real}_{j,k} an eine zentrale Einheit R übertragen. Hier wird für jeden betrachteten Sensor die Abweichung zwischen realem Messwert und Erwartungswert ΔV_{j,k} = V^{real}_{j,k} - V^{theor}_{j,k} berechnet.

Für jeden geeigneten Sensor λ wird die Häufigkeitsverteilung H{ΔV_{1,λ}, ..., ΔV_{nM,λ}} des Ausmaßes dieser Abweichung in allen Dialysegeräten berechnet. Mittels in der zentralen Einheit hinterlegter Kriterien wird nun untersucht, ob es signifikante Abweichungen für Sensor λ gibt. Treten signifikante Abweichungen nur bei wenigen Maschinen auf, so wird darauf geschlossen, dass es sich um ein isoliertes Problem an diesen Dialysegeräten, beispielsweise ein Problem eines einzelnen Aktors oder Sensors handelt. Sind die Abweichungen für die überwiegende Mehrheit der Maschinen vorhanden, so handelt es sich mit großer Wahrscheinlichkeit um ein Problem des Versorgungssystems Q. Das Ergebnis dieser Auswertung kann zentral oder durch Rückmeldung an die betroffenen Dialysegeräte angezeigt werden.

Ebenso kann eine Trendanalyse der Abweichung des Sensors λ gegenüber dem Mittelwert der übrigen, keine signifikanten Abweichungen zeigenden, Sensoren durchgeführt werden, um Langzeitdrifts des Sensors feststellen zu können. Umgekehrt können aber auch sich an der überwiegenden Mehrheit der Sensoren entwickelnde Abweichungen zur Detektion von Drifts in den Versorgungsquellen verwendet werden.

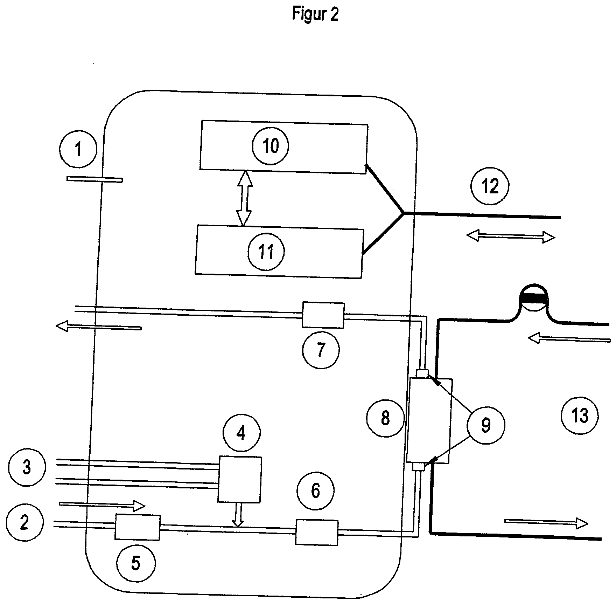

Figur 2 zeigt eine schematische Darstellung eines Dialysegerätes mit Eignung zur Anwendung in einem erfindungsgemäßen System.

Das Dialysegerät weist einen Dialysatkreislauf 13' und einen extrakorporalen Blutkreislauf 13 auf. Ein Dialysator 8, in dem der Stoffaustausch zwischen der im Dialysatkreislauf zirkulierten Dialyseflüssigkeit und dem Patientenblut stattfindet, ist an den Verbindungsstellen 9 an den Dialysatkreislauf gekoppelt. Das Dialysegerät ist an eine Stromversorgung 1 angeschlossen. Ferner ist das Dialysegerät an eine Versorgungsleitung 2 für gereinigtes Wasser (bzw. RO-Wasser) angeschlossen, wobei das gereinigte Wasser zur Herstellung der Dialyseflüssigkeit in der Mischeinheit 4 dient. Wahlweise werden ein oder mehrere Dialysekonzentrate aus einer oder mehreren Versorgungsleitungen 3 bezogen. Die Versorgungsleitungen 3 können beispielsweise an zentrale Versorgungseinheiten oder an Reservoirs angeschlossen sein. Die Aufbereitung des Dialysats aus dem gereinigten Wasser und den Konzentraten erfolgt vorzugsweise on-line in der Mischeinheit 4, welche unter anderem beispielsweise Pumpen, Leitungen, Kammern und Sensoren umfasst. Mit den Bezugszeichen 5, 6 und 7 werden Leitfähigkeitssensoren benannt, welche die temperaturkompensierte Leitfähigkeit des RO-Wassers, des frischen Dialysats stromaufwärts des Dialysators und des verbrauchten Dialysats stromabwärts des Dialysators messen. Das zentrale Versorgungssystem für mehrere derartige Dialysegeräte umfasst also im vorliegenden Beispiel eine Stromversorgung, ein System zur Bereitstellung von gereinigtem Wasser und ein System zur Bereitstellung von Dialysekonzentrat.

Um Konzentrationen der Konzentrate und in weiterer Folge der Dialyseflüssigkeit im Rahmen der erfindungsgemäßen Idee zur Erkennung von Fehlern des Versorgungssystems verwenden zu können, sollte das Dialysekonzentrat vorzugsweise aus derselben Produktionscharge stammen, auf deren Grundlage in der Auswerteeinheit R (vgl. Figur 1) der Erwartungswert V^{theor}_{j,k} als Funktion der Eigenschaften der Versorgungsquelle P^{theor}_{j,k} bestimmt wird. Die Übermittlung von Daten an die Auswerteeinheit R kann beispielsweise anhand der Verbindung 12 nach Eingabe des Benutzers am Benutzerinterface 10 des Dialysegerätes oder nach automatischer Erkennung, z.B. über Barcodeleser oder RFID erfolgen. Diese Messwerte werden an die Steuereinheit 11 des gezeigten Dialysegerätes übermittelt, welche dann die Messwerte V^{real}_{j,k} anhand der Verbindung 12 an eine zentrale Auswerteeinheit R (vgl. Figur 1) übermittelt. Anhand der Verbindung 12 können auch Daten von der zentralen Steuereinheit R empfangen werden, beispielsweise Meldungen über einen Fehler.

Neben den zum Gedanken der vorliegenden Erfindung gehörigen Funktionen übernimmt die geräteeigene Steuereinheit 11 auch bekannte Ausgaben wie die Steuerung der Dialyseprozedur durch Kontrolle der diversen Aktoren im Dialysegerät bei Verarbeitung von Benutzervorgaben aus dem Benutzerinterface 10 und der Sensormesswerte aus dem Dialysegerät.

Allen nachfolgend beschriebenen Beispielen liegt ein System zu Grunde, in dem n_{M} Dialysegeräte der in Figur 2 gezeigten Art in einem System der in Figur 1 gezeigten Art zusammengeschlossen sind.

### Beispiel 1:

Dieses Beispiel beschäftigt sich mit der Unterscheidung zwischen der Fehlfunktion eines Leitfähigkeits-Sensors und einer fehlerhaften Elektrolytkonzentration im zentralen Versorgungssystem für das Konzentrat der Dialyseflüssigkeit.

In einem hier beispielhaft aus dem System herausgegriffenen Dialysegerät wird die Dialyseflüssigkeit on-line hergestellt, wobei das Gerät das Konzentrat über die zentrale Versorgung 3 und RO-Wasser über die zentrale Versorgung 2 bezieht. Stromabwärts der Mischeinheit 4 und stromaufwärts des Dialysators 8 liegt die unverbrauchte Dialyseflüssigkeit mit der realen Zusammensetzung P^{real}_{j,k} vor, die sich aus der Mischung von Konzentrat und RO-Wasser bei der Umsetzung von Benutzervorgaben ergibt. Die temperaturkompensierte reale Leitfähigkeit V^{real}_{j,k} der unverbrauchten Dialyseflüssigkeit wird anhand des Leitfähigkeits-Sensors 6 gemessen. Der Erwartungswert für die Leitfähigkeit V^{theor}_{j,k} ergibt sich aus der angenommenen Zusammensetzung der Dialyseflüssigkeit P^{theor}_{j,k}, die sich aus den Benutzervorgaben und der bekannten Zusammensetzung des Konzentrats (z.B. Sollwerte für Natrium-Ionen und Bicarbonat-Ionen) ergibt.

Alle n_{M} Dialysegeräte senden die Werte von V^{real}_{j,k} sowie P^{theor}_{j,k} und/oder V^{theor}_{j,k} an die Auswerteeinheit R. Hier wird die Abweichung der gemessenen Leitfähigkeit von der Soll-Leitfähigkeit ΔV_{j,k} = V^{real}_{j,k} - V^{theor}_{j,k} berechnet.

Da die Erwartungswerte der Leitfähigkeit auch für unterschiedliche Benutzervorgaben bekannt sind, ist es nicht nötig, dass an allen Dialysegeräten die gleichen Einstellungen vorliegen. Um jedoch ggf. Probleme der Konzentratversorgung feststellen zu können, sollten beim Anschluss verschiedener Konzentratquellen in die Auswertung nur Daten von Maschinen mit gleicher Konzentratquelle eingehen.

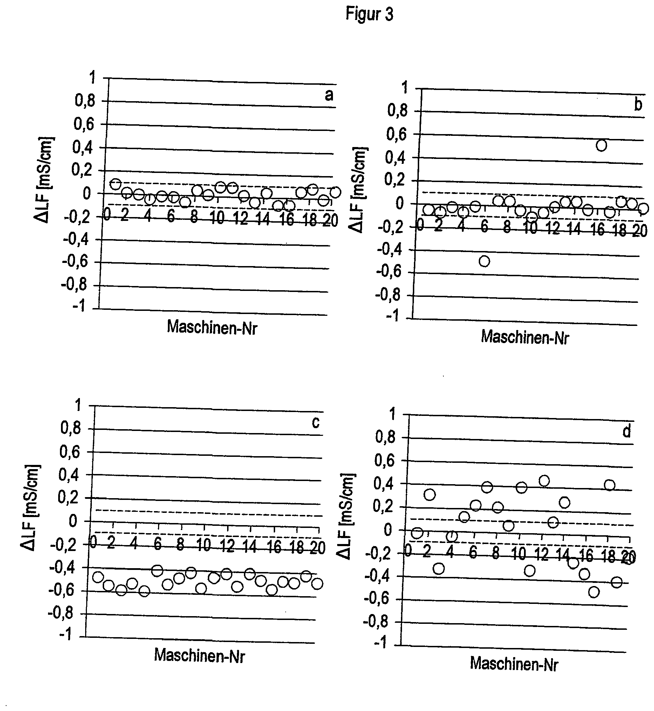

Folgende Szenarien sind möglich:
1. Bei allen Dialysegeräten ist ΔV_{j,k} (also ΔLFⱼ) kleiner als die tolerierte Abweichung, z.B. 0,1 mS/cm. Daraus folgt, dass sowohl die zentrale Konzentrat-Versorgung, als auch das gerätespezifische Mischsystem und der gerätespezifische Sensor bei allen Maschinen seine Funktion ordnungsgemäß erfüllt. Dieses Szenario ist in der Grafik gemäß Figur 3a dargestellt.
2. Bei einigen wenigen Maschinen liegt ΔLFⱼ außerhalb der tolerierten Abweichung, während bei allen anderen Maschinen die Toleranz eingehalten wird. Dieses Szenario ist in der Grafik gemäß Figur 3b dargestellt, wobei ΔLFⱼ für die Maschinen mit den Nummern 6 und 16 außerhalb der tolerierten Abweichung liegt. Daraus folgt, dass bei den Maschinen mit den Nummern 6 und 16 ein Problem im Mischsystem oder am Leitfähigkeits-Sensor vorliegt.
3. Bei allen oder zumindest bei der überwiegenden Anzahl der Maschinen liegt ΔLFⱼ außerhalb der tolerierten Abweichung, wobei Ausmaß und Richtung der Abweichung gleich sind. Dieses Szenario ist in der Grafik gemäß Figur 3c dargestellt. Daraus folgt, dass ein Problem der zentralen Konzentrat-Versorgung wahrscheinlich ist.
4. Bei allen oder zumindest bei der überwiegenden Anzahl der Maschinen liegt ΔLFⱼ außerhalb der tolerierten Abweichung, wobei Ausmaß und Richtung der Abweichung jedoch völlig unterschiedlich sind. Ein Technikereinsatz ist nötig, aber eine Diagnose der Ursache ist noch nicht möglich. Dieses Szenario ist in der Grafik gemäß Figur 3d dargestellt.

Durch eine Analyse des Langzeittrends der individuellen und kollektiven Abweichung zwischen gemessenen ΔLFⱼ und Erwartungswert sind zudem Prognosen über das Erreichen einer kritischen Größe des Messfehlers der LF-Zellen eines Dialysegeräts möglich. Ebenfalls können Drifts in der Zusammensetzung des zentralen Konzentrats (z.B. hervorgerufen durch Verdunstung) oder in der Qualität der RO (Reverse Osmose zur Bereitstellung des gereinigten Wassers) erkannt werden.

Durch entsprechende Bypass-Schaltungen (z.B. Umgehung des Dialysators 8) ist es möglich, auch weitere Leitfähigkeitssensoren in die Überwachung mit einzubeziehen. Beispielsweise kann der stromabwärts des Dialysators angeordnete Leitfähigkeitssensor 7 in die Überwachung mit einbezogen werden. Statt eines Leitfähigkeits-Sensors könnten auch Sensoren verwendet werden, die spezifisch auf einzelne Dialysat-Bestandteile empfindlich sind, z.B. ionenselektive Elektroden oder pH-Elektroden. Statt einer zentralen Konzentrat-Versorgung ist das gleiche Prinzip bei der Verwendung von Dialysekonzentrat aus einem Reservoir anwendbar, wobei hier als zusätzlicher Faktor die Chargentoleranz eingehen würde.

### Beispiel 2:

Dieses Beispiel beschäftigt sich mit der Unterscheidung zwischen der Fehlfunktion eines Leitfähigkeits-Sensors und einer fehlerhaften RO-Wasser-Versorgung.

Wie auch in Beispiel 1 wird vorliegend in einem beispielhaft aus dem System herausgegriffenen Dialysegerät die Dialyseflüssigkeit on-line hergestellt, wobei das Gerät das Konzentrat über die zentrale Versorgung 3 und RO-Wasser über die zentrale Versorgung 2 bezieht. Stromaufwärts der Mischeinheit 4 liegt RO-Wasser mit der realen Zusammensetzung P^{real}_{j,k} (Restanteil an Elektrolyten) vor. Die temperaturkompensierte reale Leitfähigkeit V^{real}_{j,k} des RO-Wassers wird anhand des Leitfähigkeits-Sensors 5 gemessen. Der Erwartungswert für die Leitfähigkeit V^{theor}_{j,k} ergibt sich aus der typischen Reinheit des für Dialysebehandlungen verwendeten RO-Wassers P^{theor}_{j,k}. Er beträgt weniger als 0,05 µS/cm.

Das Prinzip der Auswertung erfolgt analog Beispiel 1.

Das Kriterium für ein Geräte- oder Quellenproblem sind hier positive Werte von ΔLFⱼ, wobei wiederum zwischen Problemen einzelner Geräte und einem Problem der zentralen RO-Versorgung unterschieden werden kann. In gleicher Weise können an Stelle des Leitfähigkeits-Sensors 5 andere an derselben Position befindliche Sensoren zur Überwachung bestimmter Eigenschaften der RO-Versorgung eingesetzt werden, z.B. Drucksensoren zur Überwachung des Wassereingangsdrucks. Mittels Temperatursensoren kann z.B. bei einer integrierten Heißreinigung festgestellt werden, ob die mindestens erforderliche Temperatur erreicht wird, und unterschieden werden, ob ggf. bei einzelnen Geräten Probleme vorliegen (Sensorfehler, Abkühlung in zu langer Schlauchleitung) oder ob ein generelles Versagen der zentralen Reinigungsapparatur vorliegt. Die LF-Überwachung kann in gleicher Weise auch für die verschiedenen Konzentrat-Zuleitungen erfolgen.

### Beispiel 3:

Dieses Beispiel beschäftigt sich mit der Feststellung von Temperaturschwankungen am Aufstellungsort einzelner Dialysegeräte des Systems.

Die reale Umgebungstemperatur P^{real}_{j,k} = V^{real}_{j,k} wird von TemperaturSensoren S_{j,1} an der Eingangsseite eines Netzteil- oder Gehäuselüfters gemessen. Als Erwartungswert P^{theor}_{j,k} = V^{theor}_{j,k} wird die übliche Raumtemperatur herangezogen (ggf. Mittelwert der Einzelmesswerte).

Das Prinzip der Auswertung erfolgt analog Beispiel 1.

Abweichungen an einzelnen Geräten können dabei z.B. auf verstärkte Wärmeentwicklung am Gerät (Elektronik) hindeuten. Durch den Vergleich mit den anderen Geräten ist hierdurch eine empfindlichere Erkennung von Überwärmung möglich als beim Vergleich mit einer zuvor festgelegten absoluten Grenze. Wird in P^{real}_{j,k} die Position der Geräte in der Klinik mit aufgenommen, so kann eine lokale Temperaturabweichung in der Klinik (z.B. durch Sonneneinstrahlung, Fehlfunktion der Klimaanlage oder Lüftung, etc.) erkannt werden.

### Beispiel 4:

Dieses Beispiel beschäftigt sich mit der Feststellung von Fehlern in der zentralen oder gerätespezifischen Stromversorgung.

Die Versorgungsspannung P^{real}_{j,k} wird zeitaufgelöst von einen geeigneten Spannungssensor an der Spannungsversorgung 1 des Dialysegerätes gemessen. Als Messwert V^{real}_{j,k} dient der Mittelwert der gemessenen Spannung nach statistischer Auswertung oder auch die Varianz der Spannungsschwankungen. Als Erwartungswert P^{theor}_{j,k} = V^{theor}_{j,k} wird eine konstante Spannungsversorgung mit Nennwert gemäß Spezifikation des Elektrizitätswerks angenommen.

Abweichungen des Mittelwerts bzw. eine erhöhte Varianz der Versorgungsspannung an einzelnen Geräten haben als Ursache technische Probleme der Geräte selbst (z.B. Wackelkontakt), des Anschlusses (z.B. Überlastung einer Phase) oder der Messelektronik. Treten diese Probleme bei der Mehrzahl der Geräte auf, so deutet dies auf ein generelles Problem der elektrischen Versorgung hin, was gerade in technisch weniger entwickelten Regionen wie Entwicklungsländern auftreten kann.

### Beispiel 5:

Dieses Beispiel beschäftigt sich mit der Feststellung von Abweichungen von üblichen Einstellungen eines Dialysezentrums.

Als sog. "Messwerte" P^{real}_{j,k}, hier gleich V^{real}_{j,k}, dienen gemessene Dialyseparameter wie Leitfähigkeit, Temperatur, Flussraten oder dergleichen). Als Erwartungswerte V^{theor}_{j,k}, dienen die korrespondierenden zentrumsspezifisch üblichen Dialyseparameter.

Wie in Beispiel 3 wird die genaue Lage der Erwartungswerte durch den Mittelwert über die Einzelmesswerte gleicher Sensoren der verschiedenen Dialysegeräte bestimmt. Alternativ können die so bestimmten Erwartungswerte von der Recheneinheit auch gespeichert werden. Zum Vergleich kann dann der Mittelwert dieser Erwartungswerte über mehrere Behandlungstage dienen.

Abweichungen der Dialyseparameter vom Zentrumsstandard an einem der Dialysegeräte können durch Fehleingaben der Dialyseparameter durch den Anwender entstehen. Abweichungen der Messgrößen können durch Fehlfunktionen in automatischen Regelungen, z.B. des Dialysatflusses, der Blutpumpenrate, der Temperatur oder der Dialysatzusammensetzung hervorgerufen werden. In diesen Fällen kann der Benutzer zur Überprüfung seiner Einstellungen und der korrekten Funktion der automatischen Regelungen aufgefordert werden.

### Beispiel 6:

Dieses Beispiel beschäftigt sich mit der Feststellung von Abweichungen der Clearance.

Als Sensoren S_{j,option} in diesem Beispiel dienen alle zur Bestimmung der Clearance benötigten Sensoren. Für die Bestimmung der Clearance durch OCM gehören hierzu Sensoren 6 und 7 zur zeitaufgelösten Aufzeichnung der temperaturkompensierten Leitfähigkeit stromaufwärts und stromabwärts des Dialysators 8 sowie Sensoren zur Bestimmung des Blut-, Dialysat- und Substituatflusses (Bestimmung der UF-Rate).

Als reale Bedingung P^{real}_{j,OCM} an den Sensoren wird die effektive Reinigungsleistung des Dialysators für Harnstoff während der Dialyse unter Berücksichtigung von Patienteneffekten wie Rezirkulation, ausgedrückt in Dialysatorparameter (K₀A)_{effektiv} angenommen.

Die angenommene Bedingung P^{theor}_{j,OCM} am Sensor entspricht dem Dialysatorparameter (K₀A)_{effektiv} unter Annahme der für den Dialysatortyp typischen Rezirkulation (z.B. 10-15% cardiopulmonare Rezirkulation).

Messwert des Sensors V^{real}_{j,OCM} sind die Clearance bzw. der Dialysatorparameter (K₀A)_{effektiv}.

Messwert des Sensors V^{theor}_{j,OCM} sind die Clearance bzw. der Dialysatorparameter (K₀A)_{effektiv} unter Annahme der für den Dialysatortyp typischen Rezirkulation (z.B. 10-15% cardiopulmonare Rezirkulation). Alternativ wird bei Verwendung der zentrumsspezifischen Dialyseparameter (vgl. Beispiel 5) der Mittelwert aller unter gleichen Bedingungen stattfindenden Dialysen im System oder aus der Analyse historischer Behandlungen als Referenz verwendet.

OCM kann als Beispiel für einen generalisierten Sensor genommen werden, bei dem die Erlangung eines physikalischen Parameters eine komplexe Vorrichtung aus Steuereinheit und diversen Sensoren erfordert. Die Auswertung ist wie in Beispiel 1 erläutert möglich:
Eine Abweichung der Clearance unter den Erwartungswert bei wenigen Patienten deutet auf Probleme (erhöhte Rezirkulation, Clotting des Dialysators, ungünstige Einstellung der Dialyseparameter, fehlerhafte Konnektion von Patient und Dialysator) bei diesen Patienten hin. Ist dagegen die Clearance bei der überwiegenden Anzahl der Patienten reduziert, so lässt sich hieraus auf ein allgemeines Problem im Zentrum bei der aktuellen Dialyseschicht schließen. Dieses Problem könnte z.B. ein Chargenproblem der verwendeten Dialysatoren oder die Fehlbedienung des Personals (z.B. Anschluss der Dialysatoren 9 im Gleichstrom statt im Gegenstrom bei mit dem Gerätetyp nicht vertrautem Personal) sein.

## Patentansprüche

1. System umfassend mehrere medizinische Geräte (M), die an ein gemeinsames Versorgungssystem (Q) angeschlossen sind, wobei
das System eine Auswerteeinheit (R) aufweist, die mit allen Geräten (M) des Systems in Verbindung steht und so ausgebildet ist, dass in den unterschiedlichen Geräten (M) mittels eines Sensors ermittelte Messwerte, die sich auf korrespondierende Größen beziehen, zur Erkennung von Fehlern des Versorgungssystems (Q) oder eines einzelnen medizinischen Geräts (M) mit einem Erwartungswert verglichen werden,
**dadurch gekennzeichnet, dass**
die Auswerteeinheit (R) so ausgebildet ist, dass es als Fehler des Versorgungssystems (Q) identifiziert wird, wenn zu korrespondierenden Größen gehörige Werte mehrerer oder aller Geräte (M) des Systems von dem Erwartungswert abweichen, und
die Auswerteeinheit (R) ferner so ausgebildet ist, dass es als Fehler eines einzelnen Gerätes (M) identifiziert wird, wenn nur ein zu einer korrespondierenden Größe gehöriger Wert eines Geräts des Systems von dem Erwartungswert abweicht.

2. System nach Anspruch 1, **dadurch gekennzeichnet, dass** die Auswerteeinheit (R) so ausgebildet ist, dass der Erwartungswert ein Mittelwert mehrerer sich auf die korrespondierende Größen beziehender Werte ist.

3. System nach einem der vorhergehenden Ansprüche, **dadurch gekennzeichnet, dass** die Auswerteeinheit (R) so ausgebildet ist, dass eine Trendanalyse der Vergleichsdaten vorgenommen wird, anhand welcher Drifts im Versorgungssystem (Q) oder einzelner Sensoren erkannt werden.

4. System nach Anspruch 3, **dadurch gekennzeichnet, dass** die Auswerteeinheit (R) so ausgebildet ist, dass es als Drift des Versorgungssystems (Q) identifiziert wird, wenn sich Abweichungen zu korrespondierenden Größen gehöriger Werte mehrerer oder aller Geräte (M) des Systems von dem bzw. den Erwartungswerten mit der Zeit erhöhen.

5. System nach Anspruch 3, **dadurch gekennzeichnet, dass** die Auswerteeinheit (R) so ausgebildet ist, dass es als Drift eines einzelnen Gerätes (M) identifiziert wird, wenn sich die Abweichungen nur eines oder lediglich vereinzelter zu korrespondierenden Größen gehöriger Werte von dem bzw. den Erwartungswerten mit der Zeit erhöhen.

6. System nach einem der vorhergehenden Ansprüche, **dadurch gekennzeichnet, dass** es sich bei dem Gerät um eine Dialysegerät und bei dem Wert um die Leitfähigkeit der Dialyseflüssigkeit handelt, und dass der Vergleich zur Erkennung von Fehlern einer zentralen Konzentrat-Versorgung, einer zentralen Dialyseflüssigkeitsversorgung, einer zentralen Reinwasserversorgung, einer gerätespezifischen Dialyseflüssigkeits-Mischanordnung oder eines gerätespezifischen Leitfähigkeits-Sensors dient.

7. System nach einem der Ansprüche 1 bis 5, **dadurch gekennzeichnet, dass** es sich bei dem Gerät um eine Dialysegerät und bei dem Wert um die Leitfähigkeit, den Geräteeingangsdruck oder die Geräteeingangstemperatur des RO-Wassers handelt, und dass der Vergleich zur Erkennung von Fehlern einer zentralen RO-Wasser-Versorgung dient.

8. System nach einem der Ansprüche 1 bis 5, **dadurch gekennzeichnet, dass** es sich bei dem Gerät um eine Dialysegerät und bei dem Wert um den absoluten Wert oder die Varianz der Versorgungsspannung handelt, und dass der Vergleich zur Erkennung von Fehlern in der zentralen oder gerätespezifischen Stromversorgung dient.

## Claims

1. System comprising a plurality of medical devices (M), which are connected to a common supply system (Q), wherein
the system has an evaluation unit (R) which is connected to all devices (M) of the system and which is configured such that measured values (M) determined in the different devices (M) by means of a sensor, which relate to corresponding parameters are compared with an expected value for recognizing errors of the supply system (Q) and/or of an individual medical device,
**characterized in that**
the evaluation unit (R) is configured such that an error of the supply system (Q) is identified if values of a plurality of multiple devices or of all the devices (M) of the system belonging to corresponding parameters deviate from the expected value, and
the evaluation unit (R) is furthermore configured such, that an error of an individual device (M) is identified, if only one value belonging to a corresponding parameter of a device of the system deviates from the expected value.

2. System in accordance with claim 1, **characterized in that** the evaluation unit (R) is configured such, that the expected value is a mean value of a plurality of values relating to the corresponding parameters.

3. System in accordance with one of the preceding claims, **characterized in that** the evaluation unit (R) is configured so that a trend analysis of the comparison data is carried out with reference to which drifts in the supply system (Q) and/or of individual sensors are recognized.

4. System in accordance with claim 3, **characterized in that** the evaluation unit (R) is configured so that a drift of the supply system (Q) is identified if deviations of values of a plurality of devices or of all devices (M) of the system belonging to corresponding parameters increase from the expected value or values over time.

5. System in accordance with claim 3, **characterized in that** the evaluation unit (R) is configured so that a drift of an individual device (M) is identified if the deviations of only one value or of only isolated values belonging to corresponding parameters increase from the expected value or values over time.

6. System in accordance with one of the preceding claims, **characterized in that** the device is a dialysis device and the value is the conductivity of the dialysis fluid, and **in that** the comparison serves a recognition of errors of a central concentrate supply, of a central dialysis fluid supply, of a central pure water supply, of a device-specific dialysis fluid mixing arrangement or of a device-specific conductivity sensor.

7. System in accordance with one of the claims 1 to 7, **characterized in that** the device is a dialysis device and the value is the conductivity, the device input pressure or the device input temperature of the RO water; and **in that** the comparison serves the recognition of errors of a central RO water supply.

8. System in accordance with one of the claims 1 to 5, **characterized in that** the device is a dialysis device and the value is the absolute value or the variance of the supply voltage; and **in that** the comparison serves the recognition of errors in the central or device-specific power supply.

## Revendications

1. Système comprenant plusieurs appareils (M) médicaux, qui sont raccordés à un système d'alimentation (Q) commun,
le système comportant une unité d'évaluation (R), qui est en communication avec tous les appareils (M) du système et qui est configurée de telle sorte que des valeurs de mesure déterminées dans les différents appareils (M) au moyen d'un capteur et se rapportant à des grandeurs correspondantes, sont comparées avec une valeur escomptée pour détecter des erreurs du système d'alimentation (Q) ou d'un appareil (M) médical individuel,
**caractérisé en ce que**
l'unité d'évaluation (R) est configurée de telle sorte que, lorsque des valeurs associées à des grandeurs correspondantes de plusieurs ou de tous les appareils (M) du système diffèrent de la valeur escomptée, cela est identifié comme une erreur du système d'alimentation (Q), et
l'unité d'évaluation (R) est en outre configurée de telle sorte que, lorsqu'une seule valeur associée à une grandeur correspondante d'un appareil du système diffère de la valeur escomptée, cela est identifié comme une erreur d'un appareil (M) individuel.

2. Système selon la revendication 1, **caractérisé en ce que** l'unité d'évaluation (R) est configurée de telle sorte que la valeur escomptée est une valeur moyenne de plusieurs valeurs se rapportant aux grandeurs correspondantes.

3. Système selon l'une des revendications précédentes, **caractérisé en ce que** l'unité d'évaluation (R) est conçue de telle sorte qu'une analyse de tendance des données de comparaison est effectuée, au moyen de laquelle des dérives dans le système d'alimentation (Q) ou de capteurs individuels sont détectées.

4. Système selon la revendication 3, **caractérisé en ce que** l'unité d'évaluation (R) est configurée de telle sorte que, lorsque des écarts entre des valeurs associées à des grandeurs correspondantes de plusieurs ou de tous les appareils (M) du système et la ou les valeurs escomptées augmentent avec le temps, cela est identifié comme une dérive du système d'alimentation (Q).

5. Système selon la revendication 3, **caractérisé en ce que** l'unité d'évaluation (R) est configurée de telle sorte que, lorsque les écarts entre une seule valeur ou seulement des valeurs isolées associées à des grandeurs correspondantes et la ou les valeurs escomptées augmentent avec le temps, cela est identifié comme une dérive d'un appareil (M) individuel.

6. Système selon l'une des revendications précédentes, **caractérisé en ce que** l'appareil est un appareil de dialyse et la valeur est la conductibilité du liquide de dialyse, et **en ce que** la comparaison sert à détecter des erreurs d'une alimentation centrale en concentré, d'une alimentation centrale en liquide de dialyse, d'une alimentation centrale en eau pure, d'un agencement de mélange de liquide de dialyse spécifique à un appareil ou d'un capteur de conductibilité spécifique à un appareil.

7. Système selon l'une des revendications 1 à 5, **caractérisé en ce que** l'appareil est un appareil de dialyse et la valeur est la conductibilité, la pression à l'entrée de l'appareil ou la température à l'entrée de l'appareil de l'eau traitée par osmose inverse, et **en ce que** la comparaison sert à détecter des erreurs d'une alimentation centrale en eau traitée par osmose inverse.

8. Système selon l'une des revendications 1 à 5, **caractérisé en ce que** l'appareil est un appareil de dialyse et la valeur est la valeur absolue ou la variance de la tension d'alimentation, et **en ce que** la comparaison sert à détecter des erreurs de l'alimentation électrique centrale ou spécifique à un appareil.
